(19) Europäisches Patentamt
European Patent Office
Office européen des brevets

(11) Veröffentlichungsnummer: **0 036 109**
**B1**

(12) # EUROPÄISCHE PATENTSCHRIFT

(45) Veröffentlichungstag der Patentschrift: **19.10.83**

(51) Int. Cl.³: **B 01 J 29/06,** B 01 J 31/02, C 07 C 11/02, C 07 C 1/20

(21) Anmeldenummer: **81101314.3**

(22) Anmeldetag: **24.02.81**

(54) **Verfahren zur Herstellung von niederen Olefinen aus wasserhaltigem Methanol und/oder wasserhaltigem Dimethylether.**

(30) Priorität: **04.03.80 DE 3008146**

(43) Veröffentlichungstag der Anmeldung: **23.09.81 Patentblatt 81/38**

(45) Bekanntmachung des Hinweises auf die Patenterteilung: **19.10.83 Patentblatt 83/42**

(84) Benannte Vertragsstaaten: **BE DE FR GB IT NL**

(56) Entgegenhaltungen:
**EP-A-0 002 492**
**DE-A-2 615 150**
**FR-A-2 280 614**
**US-A-3 682 996**
**US-A-3 911 041**
**US-A-3 979 472**
**US-A-3 980 586**
**US-A-4 062 905**
**US-A-4 079 095**
**US-A-4 207 424**

(73) Patentinhaber: **HOECHST AKTIENGESELLSCHAFT, Postfach 80 03 20, D-6230 Frankfurt am Main 80 (DE)**

(72) Erfinder: **Wunder, Friedrich, Dr., Jahnstrasse 46, D-6093 Flörsheim am Main (DE)**
Erfinder: **Leupold, Ernst Ingo, Dr., Am Zäunefeld 15, D-6392 Neu-Anspach (DE)**
Erfinder: **Hachenberg, Horst, Dr., Mohnweg 1, D-6229 Walluf (DE)**
Erfinder: **Schmidt, Hans-Joachim, Dr., Am Burgenblick 6, D-6240 Königstein/Taunus (DE)**
Erfinder: **Unger, Klaus, Prof.Dr., Am alten Berg 40, D-6104 Seeheim-Jugenheim (DE)**

**EP 0 036 109 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von niederen Olefinen aus wasserhaltigem Methanol und/oder wasserhaltigem Dimethylether

In DE-OS 2 755 229, US-PS 4 062 905, US-PS 3 911 041, US-PS 4 079 095, US-PS 3 979 472 sowie DE-OS 2 615 150 werden Verfahren zur Herstellung von niederen Olefinen aus Methanol und/oder Dimethylether an Aluminiumsilikat-Katalysatoren beschrieben. Allen diesen Katalysatoren gemeinsam ist, daß sie periodisch regeneriert, d. h. von gebildeten Nebenprodukten befreit werden müssen, was schon bei relativ niedrigen Temperaturen von 300° C bis 500° C, am vorteilhaftesten bei der Reaktionstemperatur selbst, mit Luft erfolgen kann. Bei Einsatz von Dimethylether oder Methanol, das kein oder nur sehr wenig Wasser enthält, sind diese Katalysatoren sehr oft regenerierbar, ohne daß eine Leistungs- oder Selektivitätsminderung auftritt. Jedoch fällt dann bei nicht vollständigem Umsatz neben niederen Olefinen ein Methanol-Wassergemisch an. Das in diesem Gemisch enthaltene Methanol muß zurückgewonnen werden; bei der Destillation erhält man jedoch nur wasserhaltiges Methanol, falls man nicht einen erheblichen Aufwand treibt. Die Gegenwart von Wasser wirkt sich zwar auf die Selektivität zu niederen Olefinen günstig aus, jedoch zeigt sich, daß hierbei einige Aluminiumsilikate unter den Reaktionsbedingungen deutlich an Aktivität verlieren und nur wenige Male wieder regenerierbar sind. Eine sorgfältige Entwässerung des Methanols vor der Rückführung löst zwar dieses Problem, bedeutet jedoch einen hohen Energieaufwand.

Es bestand daher die Aufgabe, einen Aluminiumsilikat-Katalysator für die Umwandlung von wasserhaltigem Methanol und/oder wasserhaltigem Dimethylether in niedere Olefine herzustellen, der sehr oft regenerierbar ist. Überraschenderweise kann dies dadurch erreicht werden, daß man ein Aluminiumsilikat silanisiert und anschließend in Gegenwart von Sauerstoff erhitzt.

Die Silanisierung von Kieselsäuren und Silikaten ist bereits mehrfach beschrieben, z. B.: G. Erdel und K. Unger, J. Vac. Sc. Technol. 11 (1), (1974), Seite 429 oder A. V. Kiselev et al., J. Colloid. Interface Sci. 69 (1979), Seite 148.

Die Anwendung dieser Silanisierung auf Zeolithe wird in der US-PS 3 682 996 beschrieben, wobei die dadurch verminderten Absorptionseigenschaften bei Alkylierung, Kohlenwasserstoff-Crack-Reaktionen, Hydrocracken und Isomerierung zu einer erhöhten Selektivität führen. Eine anschließende Erhitzung in Gegenwart von Sauerstoff wird jedoch nicht beschrieben.

Aus der US-PS 3 980 586 ist weiter ein Verfahren zum Silanisieren von Aluminiumsilikat-Katalysatoren für Kohlenwasserstoffumwandlungen durch Behandlung mit Silanen und anschließendes Erhitzen in Gegenwart von Sauerstoff bekannt. Aus der FR-PS 2 280 614 ist die entsprechende Behandlung von $Al_2O_3$ bekannt. Es war jedoch aufgrund dieser Literaturstelle nicht vorhersehbar, daß man durch derartige Behandlung von Aluminiumsilikat einen sehr oft regenerierbaren Katalysator für die Umwandlung von wasserhaltigem Methanol und/oder wasserhaltigem Dimethylether erhält.

Es wurde nun ein Verfahren zur Herstellung von niederen Olefinen aus wasserhaltigem Methanol und/oder wasserhaltigem Dimethylether unter Verwendung von Aluminiumsilikat als Katalysator gefunden, das dadurch gekennzeichnet ist, daß das Aluminiumsilikat vor dem Einsatz silanisiert und anschließend in Gegenwart von Sauerstoff oder Sauerstoff enthaltenden Gasen auf 300 bis 600° C erhitzt wurde.

Überraschenderweise bewirkt eine Silanisierung ohne die anschließende erfindungsgemäße thermische Behandlung eine Selektivitätsabnahme, und erst durch diese anschließende Behandlung wird ein hochselektiver Katalysator erhalten, der sehr oft regenerierbar ist.

Es kommen beispielsweise die natürlichen oder synthetischen kristallinen Aluminiumsilikate in Frage, wie die unter den Bezeichnungen Faujasite, Zeolithe, Chabasite, Analcim, Gismondit, Gmelinit, Natrolith, Mordenite und Erionite bekannten Molekularsiebe. Vorteilhaft verwendet man das in großen Mengen natürlich vorkommende Chabasit-Erionitgemisch. Zum weiteren sind auch geeignet die üblichen, amorphen, sauren Crackkatalysatoren, die im allgemeinen zwischen 13 und 25 Gew.-% Aluminiumoxid und 75 bis 87 Gew.-% Kieselsäure enthalten.

Bei den amorphen wie den kristallinen Aluminiumsilikaten ist es zweckmäßig, solche mit Porendurchmessern von 0,5 nm oder höher zu verwenden.

Als Silanisierungsmittel sind alle Silane geeignet, die eine oder mehrere reaktive Gruppen tragen, welche mit Hydroxylgruppen reagieren. Bevorzugt sind Verbindungen der allgemeinen Formel

$$R_nSiX_{4-n} \qquad \text{mit } n = 1, 2, 3,$$

wobei R ein Alkylrest mit bis zu 5 C-Atomen und X = Halogen ist, sowie Verbindungen wie

Hexamethyldisilazan

$$(CH_3)_3Si-NH-Si(CH_3)_3$$

N,O-bis-Trimethylsilylacetamid

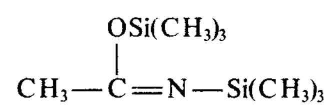

N,N-bis-Trimethylsilyltrifluoracetamid

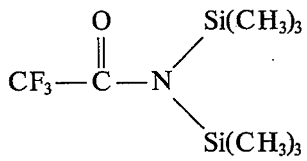

Trimethylsilylazid

$(CH_3)_3Si—N_3$

N-Trimethylsilyldiethylamin

$(CH_3)_3Si—N(C_2H_5)_2$

N-Trimethylsilylimidazol

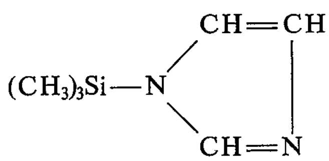

Orthokieselsäuretetramethylester

$Si(OCH_3)_4$

oder

Orthokieselsäuretetraethylester

$Si(OC_2H_5)_4$

Besonders geeignet sind die Verbindungen

$(CH_3)_3SiCl$ $(CH_3)_3SiBr$

und die reinen oder gemischten Methyl-Ethyl-Verbindungen, wie

$(C_2H_5)_3SiCl$ $(C_2H_5)_2CH_3SiCl$

$(CH_3)_2C_2H_5SiCl$

sowie die eben genannten stickstoffhaltigen Verbindungen.

Zur Herstellung der beim erfindungsgemäßen Verfahren verwendeten Katalysatoren werden die Aluminiumsilikate im allgemeinen getrocknet und dann mit gasförmigen oder flüssigen Silanen in Kontakt gebracht. Anschließend wird überschüssiges Silan entfernt, und zwar durch Auswaschen mit einem Lösungsmittel (wie Ether) oder durch Erhitzen im Vakuum. Dann wird der Katalysator durch Erhitzen auf 300° C bis 600° C, vorzugsweise 350° C bis 500° C, in Gegenwart von Sauerstoff oder einem Sauerstoff enthaltenden Gas aktiviert. Vor der Silanisierung oder nach der Aktivierung kann das Aluminiumsilikat mit Mangan beladen werden, bevorzugt durch einen Ionenaustausch, aber auch gegebenenfalls durch eine einfache Imprägnierung mit einem Mangansalz. Im Falle einer solchen Mangandotierung beträgt der Mangangehalt im allgemeinen bis zu 10%. Er bewirkt eine Steigerung der Selektivität zu niederen Olefinen.

Als Lösemittel für die Mangansalze sind Wasser, Methanol, Formamid, Dimethylformamid oder auch deren Gemische bevorzugt, insbesondere Wasser. Als Mangansalze kommen alle leicht löslichen und gut zugänglichen Salze in Frage, z. B. das Formiat, Acetat, Propionat, Butyrat, Lactat, Citrat, Tartrat, Salz der Äpfelsäure, Chlorid, Bromid, Nitrat, Sulfat.

Bei Verwendung von Molekularsieben kann eine der bei diesen Materialien üblichen Methoden der Imprägnierung mit einem Metallkation verwendet werden; das kann ein Austausch der ursprünglich auf dem Molekularsieb vorhandenen Kationen gegen Mangan sein, es kann auch eine vorgelagerte Überführung des Molekularsiebes in die acide Form sein, gefolgt von einer Behandlung mit der Lösung eines Mangansalzes.

Es hat sich weiterhin oft als vorteilhaft für eine hohe Selektivität erwiesen, noch weitere Elemente als Co-Katalysatoren zu verwenden. Als solche sind Elemente geeignet, die in ihren Verbindungen ein-, zwei- oder dreiwertig vorkommen, wie beispielsweise die Alkalimetalle (insbesondere Lithium, Natrium und Kalium), die Erdalkalimetalle (insbesondere Magnesium, Calcium und Barium), Zink, Cadmium, Lanthan, Seltene Erden (wie Praseodym, Neodym, Samarium, Gadolinium oder auch ihre Mischungen wie Didymium) und Beryllium.

Diese co-katalytisch wirksamen Metallionen können gemeinsam oder nacheinander aufgetragen werden; bei gemeinsamer Lösung muß jedoch die gegenseitige Löslichkeitsbeeinflussung berücksichtigt werden, d. h. z. B. bei Einsatz von Calcium oder Barium sind Sulfate ungeeignet.

Bei der erfindungsgemäßen Herstellung von niederen Olefinen aus Methanol unter Einsatz des Katalysators kann man entweder Methanol direkt über den Katalysator leiten (wobei das Methanol einen hohen Wasseranteil enthalten kann) oder aber es zunächst an einem üblichen Dehydrierungs-Katalysator, wie z. B. Aluminiumoxid oder Aluminiumsilikat, ganz oder teilweise in Dimethylether und Wasser überführen und den Ether dann alleine oder zusammen mit dem vorhandenen bzw. gebildeten Wasser und evtl. nicht umgesetztem Methanol über den Katalysator leiten. Man kann aber auch Gemische von Methanol und Dimethylether oder Dimethylether allein als Ausgangssubstanz verwenden.

Die Einsatzkomponenten Methanol und/oder Dimethylether können auch mit Inertgasen verdünnt in die Reaktion eingesetzt werden. Zur Erniedrigung des Partialdruckes sind beispielsweise Stickstoff und Kohlendioxid geeignet. Die Reaktion kann aber zu diesem Zweck auch bei vermindertem Druck bis herab zu 0,1 bar durchgeführt werden.

Der Wassergehalt der Einsatzprodukte kann in weiten Grenzen schwanken, bis zu etwa 80% Wasser, wobei jedoch höhere Wassermengen

höhere Verdampfungs- und Destillationskosten hervorrufen, so daß die Obergrenze des Wassergehaltes von wirtschaftlichen Überlegungen bestimmt wird.

Die Reaktionstemperatur liegt im allgemeinen zwischen 300° C und 500° C, bevorzugt zwischen 380° C und 420° C. Wählt man die Reaktionsbedingungen so, daß nur ein unvollständiger Umsatz an Methanol und/oder Dimethylether erreicht wird, so können die nicht umgesetzten Anteile abgetrennt und zurückgeführt werden. Die nach dem erfindungsgemäßen Verfahren hergestellten Alkene können nach den üblichen Methoden, z. B. durch Destillation, von den als Nebenprodukt entstehenden Alkanen und voneinander getrennt werden.

Damit steht ein Verfahren zur Verfügung, das in besonders selektiver und damit wirtschaftlicher Weise die Herstellung von industriell bedeutenden niederen Alkenen aus Methanol und/oder Dimethylether in Gegenwart von großen Mengen Wasser gestattet. Es ist im Hinblick auf den Stand der Technik besonders überraschend, daß die Silanisierung ohne anschließende aktivierende Erhitzung sich auf Leistung und Selektivität negativ auswirkt und daß die Katalysatoren erst nach einer Aktivierung mit Sauerstoff oder Sauerstoff enthaltenden Gasen bei höheren Temperaturen ihre hervorragend selektiven und stabilen Eigenschaften erhalten, obwohl die aufgebrachte Siliciummenge das Verhältnis Al : Si im Gesamtkorn nur um weniger als 0,5% verändert.

Die folgenden Beispiele erläutern das erfindungsgemäße Verfahren:

### Vergleichsbeispiel 1

(Chabasit-Erionit mit Mangan imprägniert)

Es werden 300 ml eines handelsüblichen Chabasit-Erionit-Gemisches in Form von Strangpreßlingen mit 300 ml gesättigter wäßriger Manganacetat-Lösung überschichtet, nach 48 Stunden mit Wasser ausgewaschen und getrocknet. Man erhält 202 g Katalysator mit 3,6 Gew.-% Mangan. Über diesen Katalysator werden stündlich 89,1 g Methanol bei 400° C geleitet. Man erhält 25 l Gas je Stunde, bestehend aus

26,6 Gew.-% Ethylen,
27,9 Gew.-% Propylen,
4,6 Gew.-% Butene,
6,7 Gew.-% Methan,
1,3 Gew.-% Ethan,
17,4 Gew.-% Propan,
3,0 Gew.-% Butan,
0,5 Gew.-% Sonstiges,
12,0 Gew.-% Dimethylether

sowie 9,2 g Methanol und 43,3 g Wasser. Dies entspricht einem 89,6%igen Umsatz von Methanol, einer Selektivität zu $C_2-C_4$-Olefinen von 68,9% und einer Selektivität zu $C_2-C_4$-Kohlenwasserstoffen von 93%, wenn der gebildete Dimethylether und das nicht umgesetzte Methanol zurückgeführt werden.

Bei nachlassender Leistung wird der Katalysator durch Überleiten von Luft bei 430° C regeneriert, wobei die Leistung des frischen Katalysators erreicht wird. Auch nach 26 Regenerierungen wird keine Ermüdung des Katalysators beobachtet.

### Vergleichsbeispiel 2

Man wiederholt Vergleichsbeispiel 1 mit dem einzigen Unterschied, daß dem Einsatz-Methanol Wasser zugegeben wird. Man erhält bei einem Einsatz von stündlich 87,4 g Methanol und 43,1 g Wasser 26 l eines Kohlenwasserstoff-Gases mit

30,0 Gew.-% Ethylen,
29,4 Gew.-% Propen,
5,4 Gew.-% Butene,
7,3 Gew.-% Methan,
1,2 Gew.-% Ethan,
13,2 Gew.-% Propan,
2,2 Gew.-% Butane,
0,3 Gew.-% Sonstiges,
11,3 Gew.-% Dimethylether

sowie 7,8 g Methanol und 86,0 g Wasser. Dies entspricht einem Methanol-Umsatz von 91,1%, einer Selektivität zu $C_2-C_4$-Olefinen von 74,2% und einer Selektivität zu $C_2-C_4$-Kohlenwasserstoffen von 92,5%, wenn Dimethylether und nicht umgesetztes Methanol zurückgeführt werden.

Bereits nach der (wie in Vergleichsbeispiel 1 durchgeführten) 3. Regenerierung werden nur noch Kohlenwasserstoff-Selektivitäten von 12% erreicht, d. h. daß Wasser im Einsatzmaterial den Katalysator irreversibel schädigt.

### Vergleichsbeispiel 3

Es werden 300 ml des in den Vergleichsbeispielen 1 und 2 beschriebenen Katalysators bei 200° C im Vakuum 12 Stunden getrocknet, unter Inertgasatmosphäre mit Trimethylchlorsilan überschichtet und im Rotationsverdampfer unter Rückfluß bei 50° C gekocht. Nach 6 Stunden wird das überschüssige Trimethylchlorsilan mit Dimethylether p. a. ausgewaschen und der Katalysator in einer Inertgasatmosphäre bei 120° C während 12 Stunden getrocknet. Unter den Bedingungen von Vergleichsbeispiel 2 erhält man bei einem Einsatz von stündlich 86,3 g Methanol und 44,8 g Wasser 26 l eines Kohlenwasserstoffgemisches folgender Zusammensetzung:

19,7 Gew.-% Ethylen,
24,3 Gew.-% Propylen,

7,4 Gew.-% Butene,
8,6 Gew.-% Methan,
1,7 Gew.-% Ethan,
21,8 Gew.-% Propan,
4,3 Gew.-% Butan,
0,7 Gew.-% Sonstiges,
11,6 Gew.-% Dimethylether

sowie 91 g Wasser und 0,5 g Methanol. Dies entspricht einer Selektivität zu $C_2$—$C_4$-Olefinen von 60%, einer Selektivität zu $C_2$—$C_4$-Kohlenwasserstoffen von 90,8% und einem auf Methanol bezogenen Umsatz von 99,4%.

Beispiel 1

Der wie in Vergleichsbeispiel 3 hergestellte Katalysator wird 12 Stunden bei 400°C mit Luft behandelt. Über diesen Katalysator werden unter den Bedingungen von Vergleichsbeispiel 3 stündlich 98,3 g Methanol und 53,2 g Wasser geleitet. Man erhält stündlich 23 l eines Kohlenwasserstoffgemisches folgender Zusammensetzung:

39,3 Gew.-% Ethylen,
29,7 Gew.-% Propylen,
7,8 Gew.-% Butene,
6,7 Gew.-% Methan,
1,5 Gew.-% Ethan,
6,9 Gew.-% Propan,
3,6 Gew.-% Butan,
0,8 Gew.-% Sonstiges,
3,7 Gew.-% Dimethylether

sowie 107 g Wasser und 1,8 g Methanol. Dies entspricht einem Umsatz von 98,2% bezogen auf Methanol, einer Selektivität von 81,2% zu $C_2$—$C_4$-Olefinen und einer Selektivität von 93,4% zu $C_2$—$C_4$-Kohlenwasserstoffen.

Nach 54 Regenerierungen, durchgeführt wie in Vergleichsbeispiel 1, werden praktisch unveränderte Werte erhalten.

Beispiel 2

Es werden 400 ml = 265 g eines handelsüblichen, natürlichen Chabasit-Erionitgemisches mit 2,4 Gew.-% Mangan und 1,2 Gew.-% Seltenen Erden (Gemisch) durch Ionenaustausch dotiert, bei 200°C im Vakuum 12 Stunden getrocknet, dann 24 Stunden bei 80°C dem Partialdampfdruck von Triethylchlorsilan ausgesetzt. Dann wird überschüssiges Silan im Vakuum abgedampft, anschließend wird mit Luft bei 410°C aktiviert.

Über diesen Katalysator werden bei 390°C und 1 bar stündlich 210 ml 50%iges wasserhaltiges Methanol geleitet. Man erhält 27,5 l Reaktionsgas, das folgende Zusammensetzung hat:

35,5 Gew.-% Ethylen,
38,3 Gew.-% Propen,
6,4 Gew.-% Butene (überwiegend Buten-2),
2,6 Gew.-% Methan,
0,6 Gew.-% Ethan,
4,2 Gew.-% Propan
3,4 Gew.-% Butan
0,4 Gew.-% Sonstiges,
8,6 Gew.-% Dimethylether

sowie 148 g Wasser und 4,7 g Methanol. Dies entspricht einer Selektivität von

39,1% zu Ethylen,
42,1% zu Propen,
7,0% zu Butenen.

$C_2$—$C_4$-Olefine entstehen insgesamt mit 88,2% Selektivität, $C_2$—$C_4$-Kohlenwasserstoffe mit 97,0% Selektivität, wobei Dimethylether in Methanol umgerechnet wird.

Der Katalysator zeigt nach 48 Regenerierungen eine praktisch unveränderte Leistung und Selektivität.

Beispiel 3

Es werden 250 g eines handelsüblichen natürlichen Chabasit-Erionitgemisches mit Mangan(II)-ionen ausgetauscht, gründlich ausgewaschen und bei 300°C im Vakuum 12 Stunden getrocknet. Anschließend wird mit Tetramethylkieselsäureester bei 50°C silanisiert und nach 12 Stunden der überschüssige Kieselsäureester im Vakuum entfernt. Nach einer Aktivierung von 3 Stunden in Luft bei 450°C werden 260 ml/h 50%iges wasserhaltiges Methanol bei 400°C über den Katalysator geleitet. Man erhält stündlich 35 l Reaktionsgas mit

23,5 Gew.-% Ethylen,
31,5 Gew.-% Propen,
5,6 Gew.-% Butenen,
3,9 Gew.-% Methan,
0,7 Gew.-% Ethan,
5,7 Gew.-% Propan,
2,5 Gew.-% Butan,
0,4 Gew.-% Sonstiges,
26,2 Gew.-% Dimethylether

sowie 176 g Wasser und 5,8 g Methanol. Dies entspricht einer Selektivität (unter Umrechnung von Dimethylether in Methanol) von

32,4% zu Ethylen,
43,4% zu Propen,
7,6% zu Butenen,

womit die $C_2$—$C_4$-Olefine mit zusammen 83,4% Selektivität entstehen und die $C_2$—$C_4$-Kohlenwasserstoffe mit 95,1% Selektivität. Nach 35 Regenerierungen hat der Katalysator noch die gleiche Leistung und Selektivität.

Beispiel 4

Es werden 242 g eines handelsüblichen, natürlichen Chabasit-Erionitgemisches mit Mangan ausgetauscht, im Vakuum getrocknet und in Acetonitril mit Hexamethyldisilazan silanisiert, mit reinem Acetonitril ausgewaschen, getrocknet und bei 450°C in Luft aktiviert. Der fertige Katalysator enthält 2,3 Gew.-% Mangan. Über diesen Katalysator werden bei 410°C stündlich 380 ml 66,7%iges wasserhaltiges Methanol geleitet. Man erhält 65 l eines Reaktionsgases, bestehend aus

32,5 Gew.-% Ethylen,
26,9 Gew.-% Propen,
3,5 Gew.-% Butenen,
5,4 Gew.-% Methan,
1,0 Gew.-% Ethan,
11,7 Gew.-% Propan,
1,4 Gew.-% Butan,
1,1 Gew.-% Sonstiges,
16,4 Gew.-% Dimethylether

sowie 220 g Wasser und 12,6 g Methanol. Dies entspricht einer Selektivität von

39,8% zu Ethylen,
32,9% zu Propen,
4,3% zu Butenen

(wobei Dimethylether in Methanol umgerechnet wurde), d. h., die $C_2-C_4$-Olefine entstehen zusammen mit 77% Selektivität, und die $C_2-C_4$-Kohlenwasserstoffe mit 93,5% Selektivität. Nach 49 Regenerierungen weist der Katalysator noch die gleiche Aktivität und Selektivität auf.

Beispiel 5

Es werden 167 g eines synthetischen Faujasits in Form von Strangpreßlingen mit Wasser und Kohlendioxid neutral gewaschen, anschließend mit gesättigter Manganacetat-Lösung überschichtet und nach 48 Stunden mit Wasser gewaschen, bis im Waschwasser kein Mangan mehr nachweisbar ist. Nach dem Trocknen bei 200°C im Vakuum wird der Katalysator mit Triethylorthosilikat silanisiert und anschließend bei 350°C in Luft aktiviert. Der fertige Katalysator enthält 4,6 Gew.-% Mangan.

Über diesen Katalysator werden bei 410°C je Stunde 175 ml 50%iges wasserhaltiges Methanol geleitet. Man erhält 22 l eines Reaktionsgases, das aus

18,3 Gew.-% Ethylen,
19,9 Gew.-% Propen,
12,2 Gew.-% Butenen,
11,6 Gew.-% Methan,
1,9 Gew.-% Ethan,
18,3 Gew.-% Propan,
10,6 Gew.-% Butan,
0,8 Gew.-% Sonstiges,
6,4 Gew.-% Dimethylether

besteht sowie 120 g Wasser und 5,3 g Methanol. Dies entspricht einer Selektivität (unter Umrechnung von Dimethylether in Methanol) von

19,9% zu Ethylen,
22,2% zu Propen,
13,3% zu Butenen,
11,0% zu Methan,
2,0% zu Ethan,
19,0% zu Propan,
11,2% zu Butanen,

d. h., die $C_2-C_4$-Olefine entstehen mit 55,4% Selektivität, die $C_2-C_4$-Kohlenwasserstoffe mit 87,6% Selektivität.

Nach 26 Regenerierungen haben sich Leistung und Selektivität nicht verändert.

**Patentansprüche**

1. Verfahren zur Herstellung von niederen Olefinen aus wasserhaltigem Methanol und/oder wasserhaltigem Dimethylether unter Verwendung von Aluminiumsilikat als Katalysator, dadurch gekennzeichnet, daß das Aluminiumsilikat vor dem Einsatz silanisiert und anschließend in Gegenwart von Sauerstoff oder Sauerstoff enthaltenden Gasen auf 300°C bis 600°C erhitzt wurde.

2. Verfahren nach Anspruch 1, dadurch gekennzeichnet, daß die Erhitzungstemperatur 350°C bis 500°C beträgt.

**Claims**

1. A process for the preparation of lower olefins from methanol containing water and/or dimethyl ether containing water using aluminum silicate as catalyst, characterized in that prior to use the aluminum silicate is silanazed and subsequently heated to 300°C to 600°C in the presence of oxygen or of gases containing oxygen.

2. A process as claimed in claim 1, characterized in that the heating temperature is 350°C to 500°C.

**Revendications**

1. Procédé de préparation d'oléfines inférieures à partir de méthanol aqueux et/ou d'oxyde de diméthyle aqueux, à l'aide d'un silicate d'aluminium comme catalyseur, procédé caractérisé en ce qu'avant de l'utiliser, on silane le silicate d'aluminium, puis on le chauffe en présence d'oxygène, ou de gaz contenant de l'oxygène, à une température de 300 à 600°C.

2. Procédé selon la revendication 1, caractérisé en ce que la température de chauffage est comprise entre 350 et 500°C.